(19) Europäisches Patentamt

European Patent Office

Office européen des brevets

(11) EP 0 725 054 A1

(12) EUROPÄISCHE PATENTANMELDUNG

(43) Veröffentlichungstag:
07.08.1996 Patentblatt 1996/32

(51) Int. Cl.⁶: $C07C\ 63/26$, $C07C\ 51/09$

(21) Anmeldenummer: 95120224.1

(22) Anmeldetag: 20.12.1995

(84) Benannte Vertragsstaaten:
BE DE ES GB IT LU NL

(30) Priorität: 24.01.1995 DE 19502122
02.10.1995 DE 19536814

(71) Anmelder: HÜLS AKTIENGESELLSCHAFT
D-45764 Marl (DE)

(72) Erfinder:
• Korte, Hermann-Josef, Dr.
D-45721 Haltern (DE)
• Schoengen, Anton
D-58452 Witten (DE)
• Schwarz, Christoph, Dr.
D-45768 Marl (DE)
• Jostmann, Thomas, Dr.
D-48249 Dülmen (DE)

(54) **Verfahren zur Herstellung von Terephthalsäure und ihrer Isomeren**

(57) Die Erfindung betrifft ein Verfahren zur Herstellung von Terephthalsäure (TA) aus reinem Dimethylterephthalat (DMT) und/oder DMT-Zwischenprodukt durch Hydrolyse im Gegenstromreaktor bei einem Umsatz von größer 99 % und Kristallisation zum festen Produkt, das dadurch gekennzeichnet ist, daß die Summe aus Strippdampf (S) und Reaktionswasser (W) der Beziehung

$$L \leq S + W \leq 2L,$$

genügt, wobei (L) die Wassermenge wiedergibt, die notwendig ist, um die erzeugte Terephthalsäure (TA) während der Reaktion und im Reaktorsumpf weitgehend in Lösung zu halten, und die erzeugte Terephthalsäure in einer Waschstufen-freien Kristallisation kritallisiert wird.

EP 0 725 054 A1

**Beschreibung**

Die Erfindung betrifft ein Verfahren zur Herstellung von Terephthalsäure (TA) aus reinem Dimethylterephthalat (DMT) und/oder DMT-Zwischenprodukt durch Hydrolyse im Gegenstromreaktor bei fast vollständigem Umsatz und Kristallisation zum festen Produkt.

Terephthalsäure (TA) und Dimethylterephthalat (DMT) werden großtechnisch in zahlreichen Anlagen weltweit hergestellt. DMT und TA, in den Qualitäten mit hoher Reinheit (PTA) und höchster Reinheit (PTA-p), sind wichtige Ausgangsverbindungen für die Herstellung von Polyestern. Die Anwendungsgebiete der Polyester für Fasern sowie Folien, u. a. für fotografische Filme und Magnetbänder oder Flaschen aus Polyethylenterephthalat, um nur einige zu nennen, sind lange bekannt.

Das heutige Witten-DMT-Verfahren zur Herstellung von DMT oder DMT-Zwischenprodukt beinhaltet (EP-PS 0 464 046, DE-OS 40 26 733) im wesentlichen die Verfahrensschritte

- Oxidation von para-Xylol (p-X) und para-Toluylsäuremethylester (p-TE) mit nachgeschalteter Abgasreinigung
- Veresterung der Reaktionsprodukte aus der Oxidation mit Methanol
- Trennung des entstandenen sogenannten Rohesters in

    a) eine Fraktion, die in die Oxidation zurückgeführt wird,
    b) eine Roh-DMT-Fraktion mit mehr als 99 Gew.-% DMT und
    c) eine schwersiedende Rückstandsfraktion einschließlich deren Aufarbeitung,

- Reinigung der Roh-DMT-Fraktion, beispielsweise durch Waschen, Umkristallisieren sowie Reindestillation.

Aus DMT oder aus DMT-reichen Fraktionen kann durch eine gezielte Hydrolyse in an sich bekannter Weise Terephthalsäure hergestellt werden ("Terephthalsäure und Isophthalsäure", Ullmann Bd. 22, 4. Auflage, S. 519 - 528; DE-OS 29 16 197; DE-OS 29 38 163; DE-OS 29 42 859; DE-OS 30 11 858; DE-OS 30 41 293; DE-OS 30 44 617; DE-OS 34 07 912).

So werden in diesen Verfahren reines DMT und/oder DMT-Zwischenprodukt und/oder deren Isomeren[1] (Isophthalsäure, Orthophthalsäure) durch möglichst vollständige Hydrolyse, u. a. über das Hydrolysezwischenprodukt Monomethylterephthalat (MMT) und/oder seine Isomeren, zu TA und/oder deren Isomeren umgesetzt. Als Nebenprodukte entstehen Methanol und Dimethylether.

Es ist auch bekannt, daß diese Reaktion chargenweise im Rührkesselreaktor oder kontinuierlich in einer Rührkesselkaskade durchgeführt wird, wobei das Reaktionsgleichgewicht durch Entfernen von Methanol, z. B. durch Strippen oder Destillieren, oder von TA aus der flüssigen Phase, z. B. durch Umwandlung zum Feststoff, in Richtung Produkt verschoben werden kann. Es ist Stand der Technik, die Hydrolysereaktion in einem Gegenstromreaktor mit kontinuierlicher Methanolentfernung zu betreiben.

In den herkömmlichen Verfahren werden hohe Unreinheiten an Hydrolysezwischenprodukt am Reaktorausgang in Kauf genommen, da ein nahezu vollständiger Umsatz nur mit sehr hohem Energieeinsatz, z. B. in Form von Strippdampf, zu erreichen ist. Um die handelsübliche Endreinheit im festen Produkt TA zu erreichen, muß bei den üblichen Verfahren eine aufwendige Kristallisation mit Wäsche vorgesehen werden.

In DE-PS 30 44 617 wird ein Verfahren zur Herstellung von TA durch Hydrolyse von DMT bei einem Umsatz von größer 90 % in einem Gegenstromreaktor offenbart. Dem Gegenstromreaktor ist eine mehrstufige Kristallisation, die mehrere Waschstufen einschließt, nachgeschaltet. Dieses Verfahren ist nur unter einem hohen Energieeinsatz zu betreiben, da hier neben dem Energieeinsatz zur Strippdampferzeugung erhebliche Mengen an heißem Waschwasser für die Waschstufen in der unter Druck betriebenen Kristallisation benötigt werden. Darüber hinaus ist hier der apparative Aufwand für die Kristallisation mit den dazugehörigen Waschstufen und die nachfolgende Abwasseraufarbeitung sehr hoch.

Der Erfindung lag daher die Aufgabe zugrunde, ein Verfahren bereitzustellen, das es ermöglicht, TA in einfacher und wirtschaftlicher Weise in handelsüblicher Reinheit herzustellen.

Es wurde nun gefunden, daß es möglich ist, TA bis hin zu sehr hoher Reinheit und in hervorragender Ausbeute mit einem deutlich geringeren Energieeinsatz als in vergleichbaren DMT-Hydrolyseverfahren, u. a. für die Erzeugung von Strippdampf, sowie ohne besonderen Aufwand in der Kristallisation herzustellen, d. h., auch der Aufwand für die Bereitstellung von heißem Waschwasser und die Notwendigkeit, verbrauchte Waschwassermengen aufzubereiten, können im vorliegenden Verfahren entfallen. So wurde überraschenderweise gefunden, daß die geeignete Kombination der Abstimmung der Verfahrensschritte, Hydrolyse im Gegenstromreaktor einschließlich der Strippdampferzeugung und der in einigen besonderen Ausführungsformen auf den Hydrolysereaktor aufgesetzten Methanolaufbereitung und der Kristallisation - ohne Wäsche der erzeugten TA in der Kristallisation - zu diesem besonders vorteilhaften Ergebnis führt.

---

[1] im folgenden ebenfalls gemeint, wenn DMT und seine Folgeprodukte genannt sind

Ein weiterer Vorteil, der beim vorliegenden Verfahren realisiert werden kann, ist ein nahezu geschlossener Wasserkreislauf, der sich unter anderem sehr positiv auf die Umweltverträglichkeit des Verfahrens auswirkt.

Gegenstand der vorliegenden Erfindung ist daher ein Verfahren zur Herstellung von Terephthalsäure (TA) aus reinem Dimethylterephthalat (DMT) und/oder DMT-Zwischenprodukt durch Hydrolyse im Gegenstromreaktor bei einem Umsatz von größer 99 % und Kristallisation zum festen Produkt, das dadurch gekennzeichnet ist, daß die Summe aus Strippdampf (S) und Reaktionswasser (W) der Beziehung

$$L \leq S + W \leq 2L \,,$$

genügt, wobei (L) die Wassermenge wiedergibt, die notwendig ist, um die erzeugte Terephthalsäure (TA) während der Reaktion und im Reaktorsumpf weitgehend in Lösung zu halten, und die erzeugte Terephthalsäure in einer Waschstufen-freien Kristallisation kristallisiert wird. Unter Reaktionswasser (W) soll im vorliegenden Verfahren der Wasseranteil verstanden werden, der dem Gegenstromreaktor in flüssiger Form von außen zugeführt wird, beispielsweise über den Strippdampferzeuger und/oder einen Vorreaktor.

Im allgemeinen wird die DMT-Hydrolyse im Gegenstromreaktor so durch geführt, daß man DMT und/oder DMT-haltige Fraktionen von der Kopfseite des Reaktors her sowie bis hin in den mittleren Bereich des Reaktor und Strippdampf vom unteren Teil des Reaktors her, vorzugsweise am Boden des Hydrolysereaktors, aufgibt.

Der für das vorliegende Verfahren offenbarte Gegenstromreaktor besitzt geeigneterweise 20 bis 200 Böden, vorzugsweise ist der Gegenstromreaktor mit mehr als 40 bis hin zu 200 Austauschböden ausgestattet.

Im erfindungsgemäßen Verfahren wird vorzugsweise ein Vorreaktor in Form eines Rührkessels oder einer Rührkesselkaskade oder eines Strömungsrohres verwendet, wobei die Einsatzmenge sowie das Anmischverhältnis der Edukte sowie die Fahrweise der Anlage durchaus weitgehend variabel gestaltet werden kann. Geeigneterweise schleust man hier im oder hinter dem Vorreaktor Methanol aus, das in den DMT-Prozeß zurückgeführt werden kann.

Der Gegenstromreaktor wird im allgemeinen in einem Temperaturbereich zwischen 350 und 180 °C und einem Druck, der zur Aufrechterhaltung einer flüssigen Phase im Reaktorsumpf erforderlich ist, betrieben. Geeigneterweise wird beim erfindungsgemäßen Verfahren der Reaktor mit einem Rücklauf beaufschlagt, der geeignet ist, TA im Sumpf des Reaktors in Lösung zu halten.

Das beim erfindungsgemäßen Verfahren anfallende Methanol wird vorzugsweise in der Methanolaufbereitung im Kopfteil des Reaktors aufbereitet und kann in der DMT-Anlage wieder eingesetzt werden.

Bevorzugt setzt man das Reaktionswasser aus dem erfindungsgemäßen Verfahren wieder zum Anmischen von DMT und/oder direkt in den Reaktor ein, wobei das Verhältnis Reaktionswasser (W) zu Strippdampf (S), bezogen auf das Gewicht, vorzugsweise im Bereich 1 bis 4 zu 1, besonders vorzugsweise bei 1 bis 1,5 zu 1, ganz besonders vorzugsweise bei 1 zu 1, liegt.

Das Massenverhältnis von eingesetztem Strippdampf (S) zu DMT beträgt im erfindungsgemäßen Verfahren vorzugsweise 1 zu 1 bis 6 zu 1, besonders vorzugsweise 2 zu 1 bis 4 zu 1.

In der Regel werden Brüden, die im wesentlichen aus einem Methanol-Wasser-Dampfgemisch bestehen, über Kopf aus dem Reaktor abgeführt und kondensiert.

Vorzugsweise erzeugt man im erfindungsgemäßen Verfahren bei der teilweisen oder totalen Kondensation der den Reaktor verlassenden Brüden hochwertigen Dampf, der zur Weiterverwendung, z. B. zum Einsatz in der DMT-Anlage, oder vorzugsweise als Strippdampf unter Einsatz einer Wärmepumpe, geeignet ist, wobei man die erzeugte flüssige Phase ganz oder teilweise dem Reaktor wieder zuführen kann.

Im Reaktorsumpf fällt TA als Reaktionsprodukt an, wobei man im allgemeinen bestrebt ist, daß das Produkt weitgehend in gelöster Form vorliegt. Trotzdem können bei der Hydrolyse partiell Feststoffe anfallen.

Um eine hohe bis sehr hohe Produktreinheit erzielen zu können, ist im erfindungsgemäßen Verfahren ein DMT-Umsatz von deutlich mehr als 99 % erforderlich. Es kann von Vorteil sein, bereits im Vorreaktor möglichst reines DMT für das erfindungsgemäße Verfahren einzusetzen.

Beim erfindungsgemäßen Verfahren wird durch Strippen mittels Wasserdampf der Gehalt an Monomethylterephthalat (MMT) in der wäßrigen Lösung des Reaktorsumpfes vorzugsweise auf 5 000 bis 10 Gew.-ppm, besonders vorzugsweise auf kleiner 900 bis 10 Gew.-ppm, ganz besonders vorzugsweise auf kleiner 50 Gew.-ppm, abgereichert.

Die TA-haltige wäßrige Lösung des Reaktorsumpfes wird im allgemeinen bei einer Temperatur von 350 bis 180 °C und einem Druck, der zur Aufrechterhaltung einer flüssigen Phase erforderlich ist, in eine ein- oder mehrstufige Waschstufen-freie Kristallisation überführt.

Im allgemeinen wird die Kristallisation der TA bei einer Temperatur im Bereich von 300 bis 100 °C durchgeführt. In der ein- oder mehrstufigen Kristallisation des erfindungsgemäßen Verfahrens wird Wasser vorzugsweise auf unterschiedlichen Temperaturniveaus als Kondensat ausgeschleust und dem Prozeß wieder zugeführt.

Das in der Kristallisation anfallende Wasser kann in geeigneter Weise getrennt, in den Prozeß zurückgeführt und in beliebigen Verhältnissen für die Strippdampf- und/oder Reaktionswassererzeugung eingesetzt werden.

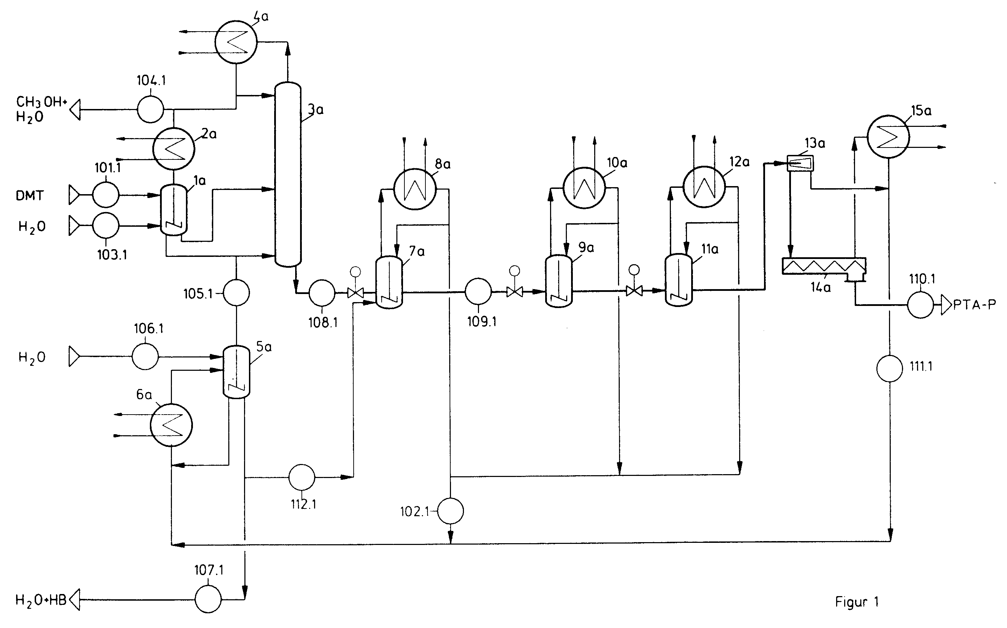

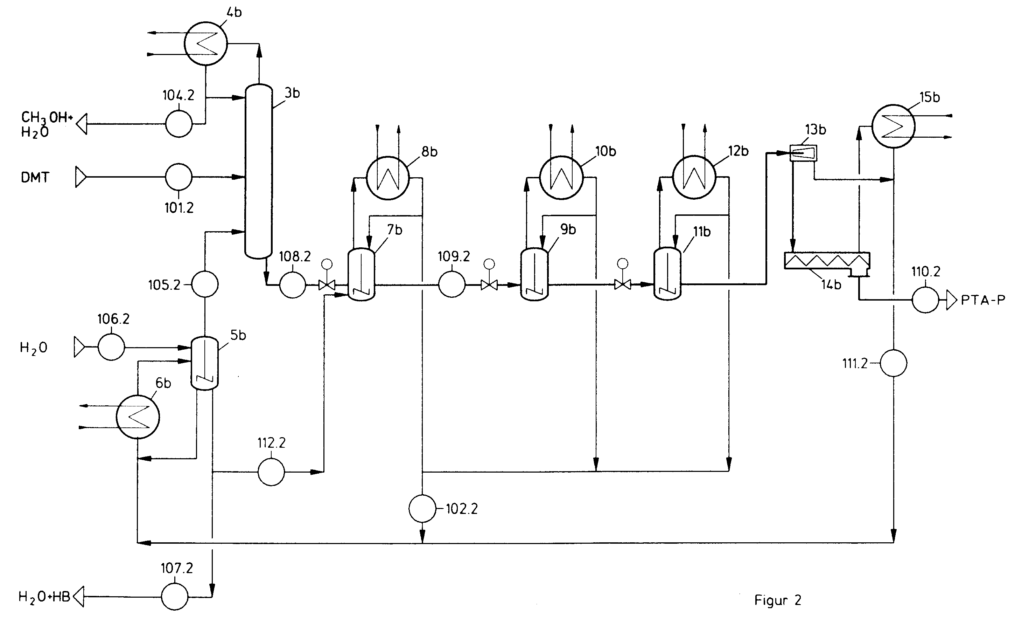

Vorzugsweise schleust man im erfindungsgemäßen Verfahren Spuren von Verunreinigungen ("High Boiler" △ HB vgl. Fig. 1 und 2) über die Strippdampferzeugung aus. Hierbei wird das Sumpfprodukt ganz oder teilweise ausgeschleust und der Rest in den Prozeß zurückgeführt.

Die in der Kristallisation des erfindungsgemäßen Verfahrens anfallende TA, d. h. PTA (Terephthalsäure hoher Reinheit) bzw. PTA-p (Terephthalsäure sehr hoher bzw. höchster Reinheit), wird in der Regel nach üblichen Trennverfahren (beispielsweise Filter, wie Band- und Trommelfilter, Druckfilter, Druckzentrifuge, ein- oder mehrstufige Dekanter, wie Druckdekanter, insbesondere Siebdekanter) und Trocknungsverfahren (beispielsweise Röhrentrockner, Stromtrockner, Wirbelbetttrockner, u. v. m.) dem Prozeß entnommen.

Die Figuren 1 und 2 zeigen Fließschemata bevorzugter Ausführungsformen des erfindungsgemäßen Verfahrens, vgl. Beispiel 1 und 2.

Isophthalsäure (ITA) sowie Orthophthalsäure (OTA) oder deren Gemische, oder deren Gemische mit TA können ebenfalls aus den jeweiligen Methylestern oder deren Gemischen, u. a. aus Dimethylisophthalat (DMI) sowie aus Dimethylorthoterephthalat (DMO), durch gezielte Hydrolyse wie im erfindungsgemäßen Verfahren erhalten werden.

Das erfindungsgemäße Verfahren weist gegenüber den bekannten Verfahren ganz besondere Vorteile auf:

- einfache Verfahrensstrukturen, die durch Wegfall von Verfahrensstufen, wie z. B. Gegenstromwäsche in der Kristallisation sowie einer vielstufigen Reaktorkaskade oder der separaten Hydrolyse-Methanoldestillation, zu vergleichsweise geringen Investitionskosten führen. Auch auf einen obengenannten Anmischreaktor als Vorreaktor kann, wenn erforderlich, verzichtet werden;
- handelsübliche bis sehr hohe Reinheiten der TA auch bezüglich MMT trotz des gegenüber Vergleichsverfahren geringeren Energieverbrauches;
- einfache Handhabung des Produktes im Reaktionsteil, zum Beispiel durch eine Fahrweise im vollständig gelösten Bereich, aber auch durch möglichen Verzicht auf Druckerhöhungspumpen im Reaktionsteil;
- geschlossener Wasserkreislauf bei minimalem Einsatz an demineralisiertem Frischwasser, dadurch kein belastetes Abwasser, geringer Energieverbrauch;
- hohe Flexibilität in bezug auf die Reinheit von TA als Funktion des Energieverbrauches;
- hoher Anpassungsgrad an Standortbedingungen durch mögliche Anpassung des gewollten Energieverbrauches über Investitionsaufwand.

Die vorliegende Erfindung wird durch die nachfolgenden Beispiele näher erläutert:

**Beispiele:**

**Beispiel 1:**

In Beispiel 1 ist eine bevorzugte Betriebsweise des erfindungsgemäßen Verfahrens wiedergegeben, bei der

- das Zusatzwasser und DMT über den Vorreaktor zugegeben werden,
- die Eindampfrate bei der Strippdampferzeugung bei rund 90 % liegt
- und keine Schwersieder aus dem Sumpfprodukt der Strippdampferzeugung ausgeschleust werden.

Das Beispiel 1 wird in einer Anlage gemäß dem Fließschema in Figur 1 ausgeführt, bei der DMT (Stoffstrom 101.1) und Zusatzwasser (Stoffstrom 103.1) über den Vorreaktor **1a** dem Reaktor **3a** zugeführt wird. Im Kondensator **4a** wird der Brüden kondensiert, ein adäquater Teil als Destillat (Stoffstrom 104.1) abgezogen und der übrige Teil der kondensierten Flüssigkeit als Rücklauf auf den Reaktor gegeben. Der im Vorreaktor erzeugte Brüden wird in Kondensator **2a** kondensiert und zusammen mit dem übrigen Destillat ausgeschieden (Stoffstrom 104.1). Das gesamte Reaktionsmethanol ist in Stoffstrom 104.1 enthalten und wird in die Methanoldestillation der DMT-Anlage gefahren. Von dort aus wird das im Stoffstrom 104.1 enthaltene Wasser mit dem Stoffstrom 103.1 wieder in den Hydrolyseprozeß zurückgeführt. Der Stoffstrom 103.1 enthält ebenfalls das bei der Hydrolyse angelagerte Wasser sowie etwaige Wassermengen, die in ausgeschleusten Produktströmen enthalten sind. In Beispiel 1 wird der Stoffstrom 107.1 mit 0 kg/h eingesetzt. Sonstiges Wasser (Stoffstrom 106.1) wird nicht zugeführt. Im Verdampfer **5a** mit Wärmetauscher **6a** wird der Strippdampf (Stoffstrom 105.1) erzeugt. Bemerkenswert ist, daß sich MMT unter den bei der Strippdampferzeugung geltenden Bedingungen nahezu vollständig zu TA umsetzt. Das Sumpfprodukt des Verdampfers **5a** wird mit Stoffstrom 112.1 in den Kristallisator **7a** geleitet, in dem auch das im Reaktor **3a** erzeugte Reaktionsprodukt (Stoffstrom 108.1) entspannt wird. In Kondensator **8a** wird der entstehende Brüden kondensiert und wieder in den Behälter **7a** zurückgefahren. Die Rührbehälter **9a** und **11a** mit den Kondensatoren **10a** und **12a** stellen weitere Abkühl- bzw. Kristallisationsstufen dar. Bemerkenswert ist, daß unter den hier vorherrschenden Kristallisationsbedingungen sich in der fertigen PTA ein MMT-Gehalt in % oder Gew.-ppm einstellt, der bei einem Viertel des Wertes in der Flüssigphase liegt, solange der Wert am Ausgang des Reaktors bei unter 1 000 ppm liegt. Die in der Kristallisation entstandene Kristallsuspension wird in Dekanter **13a** getrennt. Die feste Phase wird in Trockner **14a** von der noch anhaftenden Restfeuchtigkeit befreit und als feste TA ausgeschleust, wobei die erhaltene Qualität hochreiner PTA-p entspricht. Der Brüden der Trocknung wird in Kondensator **15a** kondensiert und zusammen mit dem Filtrat der Zentrifuge **13a** als Stoffstrom 111.1 in den Strippdampferzeuger **5a** gepumpt. Im vorliegenden Beispiel ist der Wasserkreislauf über den Prozeß geschlossen.

Die Temperaturen und die Mengenangaben zu den Stoffströmen - einschließlich der hierin enthaltenen Komponenten - aus Beispiel 1 sind in Tabelle 1 zusammengestellt, wobei sich die Mengenangaben jeweils auf einen Stoffstrom von 1000 kg TA pro Stunde beziehen.

**Beispiel 2:**

Das Beispiel 2 wird in einer Anlage gemäß dem Fließschema in Figur 2 durchgeführt. Figur 2 zeigt ebenfalls eine bevorzugte Ausführungsform des erfindungsgemäßen Verfahrens, wobei hier auf einen Vorreaktor mit Kondensator verzichtet wird. Das DMT (Stoffstrom 101.2) wird im vorliegenden Beispiel direkt in den Reaktor **3b** gefahren. Das gesamte Zusatzwasser wird hier über Stoffstrom 106.2 in den Verdampfer **5b** mit Wärmetauscher **6b** gegeben. Außerdem wird über den Stoffstrom 107.2 eine kleine Menge ausgeschleust, die auch Hochsieder, anfallende Fremdpartikel sowie einen zur problemlosen weiteren Handhabung notwendigen, aber vergleichsweise geringen Wasseranteil enthält. Die im Strom 107.2 enthaltenen Stoffe werden also nicht in die Hydrolyse zurückgegeben, wodurch sich die Mengen an eingesetztem DMT (Stoffstrom 101.2) sowie Wasser (Stoffstrom 106.2) gegenüber Beispiel 1 geringfügig erhöhen. Darüber hinaus wird über den Stoffstrom 112.2 des vorliegenden Beispiels kein Sumpfprodukt von Verdampfer **5b** in den Prozeß zurückgeführt. Durch diese Fahrweise wird der Fremdpartikelanteil in der PTA-p erheblich reduziert, wodurch sich die Qualität der erzeugten TA nochmals erheblich verbessert. Die Strippdampferzeugung ist bei dieser Fahrweise etwas erhöht. Im übrigen entspricht die Beschreibung von Figur 2 und die damit verbundene Fahrweise im Prinzip der von Figur 1 bzw. der aus Beispiel 1.

Die Temperaturen und die Mengenangaben zu den Stoffströmen - einschließlich der hierin enthaltenen Komponenten - aus Beispiel 2 sind der Tabelle 2 zu entnehmen, wobei sich die Mengenangaben jeweils auf einen Stoffstrom von 1000 kg TA pro Stunde beziehen.

**Legende zu Figur 1:**

Fließschema einer bevorzugten Ausführungsform des erfindungsgemäßen Verfahrens

| | |
|---|---|
| **1a :** | Vorreaktor |
| **2a :** | Kondensator |
| **3a :** | Gegenstromreaktor |
| **4a :** | Kondensator, aufgesetzt |
| **5a :** | Verdampfer |
| **6a :** | Wärmetauscher |
| **7a, 9a, 11a :** | Kristallisationsbehälter, rührbar |
| **8a, 10a, 12a:** | Kondensatoren |
| **13a :** | Dekanter |
| **14a :** | Trockner |
| **15a :** | Kondensator |

**Legende zu Figur 2:**

Fließschema einer weiteren bevorzugten Ausführungsform des erfindungsgemäßen Verfahrens

| | |
|---|---|
| **3b :** | Gegenstromreaktor |
| **4b :** | Kondensator, aufgesetzt |
| **5b :** | Verdampfer |
| **6b :** | Wärmetauscher |
| **7b, 9b, 11b :** | Kristallisationsbehälter, rührbar |
| **8b, 10b, 12b:** | Kondensatoren |
| **13b :** | Dekanter |
| **14b :** | Trockner |
| **15b :** | Kondensator |

**Legende der Abkürzungen:**

| | |
|---|---|
| p-X : | para-Xylol |
| p-TA : | para-Toluylsäure |
| p-TE : | para-Toluylsäuremethylester (pT-Ester) |
| HM-BME : | Hydroxymethylbenzoesäuremethylester |
| MM-BME : | Methoxymethylbenzoesäuremethylester |
| DMT : | Dimethylterephthalat |
| MMT : | Monomethylterephthalat (Terephthalsäuremonomethylester) |
| TA : | Terephthalsäure |
| MTA : | mittelreine Terephthalsäure |
| PTA : | Terephthalsäure hoher Reinheit |
| PTA-p : | Terephthalsäure sehr hoher, d. h. höchster Reinheit (Gehalt an MMT und p-TA von zusammen < 50 Gew. -ppm) |
| TAS : | Terephthalaldehydsäure (4-CBA) |
| TAE : | Terephthalaldehydsäuremethylester |
| DME : | Dimethylether |
| DMI : | Dimethylisophthalsäure |
| DMO : | Dimethylorthophthalsäure |
| ITA : | Isophthalsäure |
| OTA : | Orthophthalsäure |

**Tabelle 1:**

Aufstellung der Temperaturen und der Gehalte an Komponenten der Stoffströme aus Beispiel 1, vgl. Figur 1. Die Mengen sind in kg angegeben und jeweils auf einen Stoffstrom von 1000 kg TA pro Stunde bezogen.

| Stoffstrom | 101,1 | 102,1 | 103,1 | 104,1 | 105,1 | 106,1 | 107,1 | 108,1 | 109,1 | 110,1 | 111,1 | 112,1 |
|---|---|---|---|---|---|---|---|---|---|---|---|---|
| Temperatur [°C] | 160 | - | 100 | 220 | 270 | - | - | 265 | 200 | 100 | 100 | 270 |
| Komponenten [kg] | | | | | | | | | | | | |
| DMT | 1168,63 | - | - | - | - | - | - | - | - | - | - | - |
| TA | - | - | - | - | - | - | - | 999,69 | 1002,94 | 999,94 | 3,0 | 3,29 |
| $H_2O$ | - | - | 345,39 | 128,52 | 2699,98 | - | - | 2700,0 | 3000,0 | - | 3000,0 | 300 |
| $CH_3OH$ | - | - | - | 385,55 | 0,349 | - | - | 0,3 | 0,3 | - | 0,3 | - |
| MMT | - | - | - | - | - | - | - | 0,3 | 0,3 | 0,025 | 0,275 | - |
| TAE/4-CBA | 0,011 | - | - | - | - | - | - | 0,01 | 0,01 | 0,01 | - | - |
| DMI/IPA | 0,029 | - | - | - | - | - | - | 0,025 | 0,3 | 0,025 | 0,275 | 0,275 |
| Σ Summe | 1168,670 | 0 | 345,39 | 514,07 | 2700,33 | 0 | 0 | 3700,625 | 4003,85 | 1000,0 | 3003,85 | 303,52 |

EP 0 725 054 A1

**Tabelle 2:**

Aufstellung der Temperaturen und der Gehalte an Komponenten der Stoffströme aus Beispiel 2, vgl. Figur 2. Die Mengen sind in kg angegeben und jeweils auf einen Stoffstrom von 1000 kg TA pro Stunde bezogen.

| Stoffstrom | 101,2 | 102,2 | 103,2 | 104,2 | 105,2 | 106,2 | 107,2 | 108,2 | 109,2 | 110,2 | 111,2 | 112,2 |
|---|---|---|---|---|---|---|---|---|---|---|---|---|
| Temperatur [°C] | 160 | - | - | 220 | 270 | 100 | 270 | 265 | 200 | 100 | 100 | - |
| Komponenten [kg] | | | | | | | | | | | | |
| DMT | 1172,4 | - | - | - | - | - | - | - | - | - | - | - |
| TA | - | - | - | - | - | - | 3,25 | 1002,94 | 1002,94 | 999,94 | 3,0 | - |
| $H_2O$ | - | - | - | 128,96 | 3346,55 | 376,58 | 30,0 | 3000,0 | 3000,0 | - | 3000,0 | - |
| $CH_3OH$ | - | - | - | 386,88 | 0,35 | - | - | 0,3 | 0,3 | - | 0,3 | - |
| MMT | - | - | - | - | - | - | - | 0,3 | 0,3 | 0,025 | 0,275 | - |
| TAE/4-CBA | 0,0109 | - | - | - | - | - | - | 0,01 | 0,01 | 0,01 | - | - |
| DMI/IPA | 0,35 | - | - | - | - | - | 0,275 | 0,3 | 0,3 | 0,025 | 0,275 | - |
| Σ Summe | 1172,76 | 0 | 0 | 515,78 | 3346,9 | 376,58 | 33,53 | 4003,85 | 4003,85 | 1000,0 | 3003,85 | 0 |

8

EP 0 725 054 A1

**Patentansprüche**

1. Verfahren zur Herstellung von Terephthalsäure (TA) aus reinem Dimethylterephthalat (DMT) und/oder DMT-Zwischenprodukt durch Hydrolyse im Gegenstromreaktor bei einem Umsatz von größer 99 % und Kristallisation zum festen Produkt,
   dadurch gekennzeichnet,
   daß die Summe aus Strippdampf (S) und Reaktionswasser (W) der Beziehung

   $$L \leq S + W \leq 2L ,$$

   genügt, wobei (L) die Wassermenge wiedergibt, die notwendig ist, um die erzeugte Terephthalsäure (TA) während der Reaktion und im Reaktorsumpf weitgehend in Lösung zu halten, und die erzeugte Terephthalsäure in einer Waschstufen-freien Kristallisation kritallisiert wird.

2. Verfahren nach Anspruch 1,
   dadurch gekennzeichnet,
   daß der Gegenstromreaktor mit mehr als 20 bis hin zu 200 Austauschböden ausgestattet ist.

3. Verfahren nach Anspruch 2,
   dadurch gekennzeichnet,
   daß der Gegenstromreaktor mit mehr als 40 bis hin zu 200 Austauschböden ausgestattet ist.

4. Verfahren nach mindestens einem der Ansprüche 1 bis 3,
   dadurch gekennzeichnet,
   daß durch Strippen mittels Wasserdampf der Gehalt an Monomethylterephthalat (MMT) in der wäßrigen Lösung des Reaktorsumpfes auf 5 000 bis 10 Gew.-ppm abgereichert wird.

5. Verfahren nach Anspruch 4,
   dadurch gekennzeichnet,
   daß durch Strippen mittels Wasserdampf der Gehalt an Monomethylterephthalat (MMT) in der wäßrigen Lösung des Reaktorsumpfes auf kleiner 900 bis 10 Gew.-ppm abgereichert wird.

6. Verfahren nach Anspruch 5,
   dadurch gekennzeichnet,
   daß durch Strippen mittels Wasserdampf der Gehalt an Monomethylterephthalat (MMT) in der wäßrigen Lösung des Reaktorsumpfes auf kleiner 50 Gew.-ppm abgereichert wird.

7. Verfahren nach mindestens einem der Ansprüche 1 bis 6,
   dadurch gekennzeichnet,
   daß man das Reaktionswasser zum Anmischen von DMT und/oder direkt in den Reaktor einsetzt, wobei das Verhältnis Reaktionswasser (W) zu Strippdampf (S), bezogen auf das Gewicht, im Bereich 1 bis 4 zu 1 liegt.

8. Verfahren nach Anspruch 7,
   dadurch gekennzeichnet,
   daß man das Reaktionswasser zum Anmischen von DMT und/oder direkt in den Reaktor einsetzt, wobei das Verhältnis Reaktionswasser (W) zu Strippdampf (S), bezogen auf das Gewicht, im Bereich 1 bis 1,5 zu 1 liegt.

9. Verfahren nach Anspruch 8,
   dadurch gekennzeichnet,
   daß man das Reaktionswasser zum Anmischen von DMT und/oder direkt in den Reaktor einsetzt, wobei das Verhältnis Reaktionswasser (W) zu Strippdampf (S), bezogen auf das Gewicht, bei 1 zu 1 liegt.

10. Verfahren nach mindestens einem der Ansprüche 1 bis 9,
    dadurch gekennzeichnet,
    daß das Massenverhältnis von eingesetztem Strippdampf (S) zu DMT 1 zu 1 bis 6 zu 1 beträgt.

11. Verfahren nach Anspruch 10,
    dadurch gekennzeichnet,
    daß das Massenverhältnis von eingesetztem Strippdampf (S) zu DMT 2 zu 1 bis 4 zu 1 beträgt.

**12.** Verfahren nach mindestens einem der Ansprüche 1 bis 11,
dadurch gekennzeichnet,
daß der Gegenstromreaktor mit einem Rücklauf beaufschlagt wird, der geeignet ist, TA im Sumpf des Reaktors in Lösung zu halten.

**13.** Verfahren nach mindestens einem der Ansprüche 1 bis 12,
gekennzeichnet durch
einen Vorreaktor in Form eines Rührkessels oder einer Rührkesselkaskade oder eines Strömungsrohres.

**14.** Verfahren nach mindestens einem der Ansprüche 1 bis 13,
dadurch gekennzeichnet,
daß man Methanol im oder hinter dem Vorreaktor ausschleust.

**15.** Verfahren nach mindestens einem der Ansprüche 1 bis 14,
dadurch gekennzeichnet,
daß man bei der teilweisen oder totalen Kondensation der den Reaktor verlassenden Brüden hochwertigen Dampf erzeugt, der zur Weiterverwendung, zum Einsatz in der DMT-Anlage oder als Strippdampf unter Einsatz einer Wärmepumpe, geeignet ist, wobei man die erzeugte flüssige Phase ganz oder teilweise dem Reaktor wieder zuführt.

**16.** Verfahren nach mindestens einem der Ansprüche 1 bis 15,
dadurch gekennzeichnet,
daß das in der Kristallisation anfallende Wasser getrennt, in den Prozeß zurückgeführt und in beliebigen Verhältnissen gemeinsam für die Strippdampf- und/oder Reaktionswassererzeugung eingesetzt wird.

**17.** Verfahren nach mindestens einem der Ansprüche 1 bis 16,
dadurch gekennzeichnet,
daß man Spuren von Verunreinigungen über die Strippdampferzeugung ausschleust.

**18.** Verfahren nach mindestens einem der Ansprüche 1 bis 17,
dadurch gekennzeichnet,
daß man in der ein- oder mehrstufigen Kristallisation Wasser auf unterschiedlichen Temperaturniveaus ausschleust und dem Prozeß wieder zuführt.

**19.** Verfahren nach mindestens einem der Ansprüche 1 bis 18,
dadurch gekennzeichnet,
daß man das anfallende Methanol aufarbeitet und es mit geringen Verlusten wieder in der DMT-Anlage als Rohstoff einsetzt.

**20.** Verfahren nach mindestens einem der Ansprüche 1 bis 19,
dadurch gekennzeichnet,
daß bei der Hydrolyse partiell Feststoffe anfallen.

Figur 1

Figur 2

EP 0 725 054 A1

**Europäisches Patentamt**

# EUROPÄISCHER RECHERCHENBERICHT

Nummer der Anmeldung

EP 95 12 0224

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.6) |
|---|---|---|---|
| D,Y | DE-A-30 44 617 (DYNAMIT NOBEL AG)<br>* Seite 7, Zeile 23 - Seite 8, Zeile 13 *<br>* Ansprüche 1-10 *<br>--- | 1 | C07C63/26<br><br>C07C51/09 |
| Y | DE-A-16 18 503 (HERCULES INC.)<br>* Ansprüche 1-8 *<br>--- | 1 | |
| Y | US-A-4 578 510 (DOERR)<br>* Anspruch 1 *<br>----- | 1 | |

RECHERCHIERTE SACHGEBIETE (Int.Cl.6)

C07C

Der vorliegende Recherchenbericht wurde für alle Patentansprüche erstellt

| Recherchenort | Abschlußdatum der Recherche | Prüfer |
|---|---|---|
| DEN HAAG | 26.April 1996 | Klag, M |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P04C03)